# EUROPEAN PATENT APPLICATION

(11) **EP 2 317 301 A2**
(43) Date of publication of application: **04.05.2011**
(21) Application number: 10189653.8
(22) Date of filing: 02.11.2010
(51) Int. Cl.: G01N 1/40

(54) **Analysis kit for analysing pesticides**

(30) Priority: 02.11.2009 NL 2003742
(71) Applicant: TNO Bigg AgriQ B.V., 6709 Wageningen (NL)
(72) Inventor: van der Meer, Reinder Ronald Alexander, 6703 EL, Wageningen (NL)
(74) Representative: van Westenbrugge, Andries

(57) **Abstract**

The present invention provides a method for preparing a fruit sample for analysis. The method comprises (a) providing a ground vegetable sample to a container volume of a container, wherein the container volume or a closure therefore comprise a closed compartment containing an analytical liquid, wherein the closed compartment is configured to provide the analytical liquid into the container volume upon activation; (b) closing the container volume with the closure; and (c) opening the closed compartment and mixing the fruit sample and the analytical liquid. The container can, if desired, be shipped to a specialized laboratory, whereas possible decay of the sample may be of no influence on the solved pesticides. Further, a much smaller sample can be sent to the specialized laboratory.

## Description

### Field of the invention

The invention relates to a method for preparing a sample, especially a vegetable sample, even more especially a mashed fruit, wherein the prepared sample can be used for analysis. The invention further relates to a specific container that can be used to contain the sample, to a transport unit, as well as to an analysis kit.

### Background of the invention

Pesticide extraction kits are known in the art. For instance, US2009214734 describes a method of extracting residual pesticides in an agricultural product and a kit to be used therein. Namely, a method of extracting residual pesticides which comprises: (1) a step of processing an agricultural product into a form allowing the extraction of the residual pesticides; (2) a step of treating the thus processed agricultural product with a dehydrating agent; and (3) a step of extracting the residual pesticides from the thus dehydrated agricultural product by using a hydrophobic solvent, which has an octanol/water partition coefficient of from 0 to 4, or a mixed solvent of hydrophobic solvent and hydrophobic solvent. Further, this document describes a kit to be used in the method as described above. By using the above method and kit, the procedure can be simplified and an effect of reducing the amount of extracted contaminants such as pigments can be established.

US4505433 describes a device for grinding and transporting solids, such as surgical tissue samples, is disclosed which comprises in a preferred form a container having an upper portion defining an upwardly open top and a grinder receivable in the interior of the container. The grinder has a grinding head having a shape closely conforming to the shape of the container interior bottom, a cap that is screwed onto the upper portion of the container, and a compressible intermediate and body section joining the grinding head and the grinder top. A reservoir containing bacterial maintenance fluid, such as a frangible glass ampoule containing such fluid, is carried within the compressible intermediate section. The grinding device has a grinding configuration wherein the grinding head is received in the bottom interior of the container with the cap adjacent the container upper portion. In use, surgical tissue is placed in the container and the device is placed in the grinding configuration. Grinding is effected through application of the cap to the container upper portion to close the container, the cap forcing the grinding head into the bottom of the container interior and rotating it therein, thereby grinding tissue between the grinding head and the interior bottom. Compression of the intermediate portion of the grinder through the application of the cap to the container further places the glass ampoule into compression and then breaks it, releasing the bacterial maintenance fluid to keep the tissue moist and reduce any molecular oxygen present.

W02006127098 describes methods for determining the iron content of an in-use lubricant, which may be used on-site are disclosed. Test kits for conducting the methods are also described. In one embodiment, a method comprises: adding a predetermined amount of said in-use lubricant to an active solvent comprising predetermined amounts of an apolar organic solvent, a polar organic solvent, an organic acid, water, an iron complexing agent and a reducing agent, said active solvent having a pH between 2 and 4; thoroughly mixing the lubricant and the active solvent until the lubricant is completely dissolved; allowing the mixture to react completely and separate into a top layer and a bottom layer, the bottom layer comprising an iron complex; filtering at least a portion of the bottom layer of the mixture directly into a receptacle suitable for absorbance measurement in the visible range; photometrically measuring the net absorbance of the filtered solution at frequencies in the visible range; and converting the absorbance measurement to ppm iron content of the lubricant.

### Summary of the invention

A disadvantage of prior art methods is that those methods do not solve the problem of reliably providing a sample that can easily be transported to an analysis lab remote from the place where the sample is taken. Further, present day analysis methods of fruit and vegetables often include transport of the whole fruit or vegetable, respectively, which implies a large volume to provide a reliable amount of sample, and which may imply problems with respect of conservation of the material.

Hence, it is an aspect of the invention to provide an alternative method for preparing a sample, such as a fruit sample, a vegetable sample, a water sample, a soil sample, or plant material sample, or any other sample (see also below) which preferably further at least partly obviates one or more of above-described drawbacks.

In a first aspect, the invention provides a method for preparing a sample, such as a sample selected from the group consisting of a fruit sample, a vegetable sample, a water sample, a soil sample, or a plant material sample, for analysis, the method comprising:
a. providing a (ground) sample to a container volume of a container, wherein the container volume or a closure therefore (preferably) comprise a closed compartment containing an analytical liquid, wherein the closed compartment is configured to provide the analytical liquid into the container volume upon activation;
b. closing the container volume with the closure; and
c. (preferably) opening the closed compartment and mixing the (ground) sample and the analytical liquid.

Especially when the sample is a sample selected from the group consisting of a fruit sample, a vegetable sample and a plant material sample, the sample in the container volume of the container is preferably a mashed sample. Such samples are considered to be part of the group of vegetable samples. Hence, the term vegetable (here noun) may mean an edible plant or part of a plant other than a sweet fruit or seed, but herein the term vegetable sample, wherein vegetable is used as adjective, refers to any plant related samples, including from fruit, vegetables and seeds. Hence, when here "vegetable sample" is used next to "fruit sample", the term vegetable sample especially refers to an edible plant or part of a plant other than a sweet fruit or seed, but wherein it is referred to "vegetable sample" in general, it refers to any plant or part of a plant, such as (a part of) a fruit, vegetable (like lettuce, endive, etc.), carrots, maize, etc. The term "plant sample", when used next to "fruit sample" and "vegetable sample" may refer to those plants or plant parts, that may not be considered fruit or vegetable, such as part of a stem of a fruit plant, or a plant (part) not being a fruit plant (part) or vegetable (part).

Hence, according to a further embodiment, the method further involves mashing (or grounding) the sample, such as a fruit sample, a vegetable sample or a plant material sample, and introducing the mashed sample in the container volume. In yet another embodiment, the sample is mashed (or ground) while residing in the container volume. Hence, according to a further aspect, the invention involves providing the sample to the container and mashing the sample, especially for those embodiments wherein the sample is selected from the group consisting of a fruit sample, a vegetable sample and a plant material sample.

The term "prepared sample" refers to the mixture of sample and analytical liquid. For instance, when mixing the ground vegetable sample and analytical liquid, a prepared vegetable sample is obtained.

Therefore, in a specific embodiment the invention provides a method for preparing a sample, such as selected from the group consisting of a fruit sample, a vegetable sample, and a plant material sample, for analysis, comprising:
a. providing a ground, such as a mashed, sample to a container volume of a container, wherein the container volume or a closure therefore comprise a closed compartment containing an analytical liquid, wherein the closed compartment is configured to provide the analytical liquid into the container volume upon activation;
b. closing the container volume with the closure; and
c. opening the closed compartment and mixing the sample (i.e. the ground, such as a mashed, (fruit) sample, or other sample) and the analytical liquid.

Samples that can be introduced into the container volume are for instance (mashed) samples from apples, bananas, oranges, kiwis, from corn, maize, potatoes, coffee beans, other type of beans, such as kidney beans, navy beans, etc., from lettuce, carrots, processed food products, milk, cabbage, cherries, herbs, mangoes, vegetables, etc. Samples can however also be water or soil. For instance, from 1 kg apples, compote can be made, of which part can be filled into the container volume. Other samples that may be introduced may be selected from the group consisting of fish (including shrimps, etc.), meat, urine, tissue (human body tissue, or animal body tissue, or plant body tissue), tobacco, etc.

Those samples, mashed or not, can be provided to the container. The container may have an indication to which the container has to be filled, in order to obtain reliable and comparable sample amounts. Alternatively or additionally, by weighing the container (such as before and after filling), the predetermined amount of filling can be estimated and controlled.

Herein, the term "ground vegetable sample" is applied. As indicated above, the term "vegetable sample" refers to plant or part of a plant. Hence, a vegetable sample may be a plant or its fruit or its root or its leaves. For instance, the vegetable sample may be an apple, a banana, an orange, a kiwi, corn, maize, a potato, a coffee bean, another type of beans, such as a kidney bean, a navy bean, etc., a lettuce, a carrot, a cabbage, a cherry, a herb, mango, etc., or a ground sample thereof. Grinding may be done by cutting, slicing, etc., and may for instance be performed with a blender or grinder. By grinding, a ground sample is obtained, which may dependent upon the type of sample be a mashed sample, such as in the case of many type of fruits. It may be a powder, for instance in the case of corn or maize. In a specific embodiment, the ground vegetable sample is a mashed fruit sample. Herein, the invention is especially described with reference to mashed fruit samples, but as indicated above, the invention may also be applied to other types of samples, such as any ground vegetable sample, or even other samples.

Grinding also facilitates the provision of a homogeneous sample. By mashing apples, a homogenous sample (mousse or compote) may be obtained. Likewise, by grinding maize, a homogeneous powder may be obtained.

The phrase "container volume or a closure therefore" indicates that the closure is especially configured to be used as closure to the container. Hence, by arranging the closure to the container volume, the container volume is substantially closed.

The container is closed, and preferably locked against undesired opening to avoid cross contamination and/or leakage. Preferably, the container is sealed or is provided with a seal.

The method of the invention may be used to fill the container volume with a predetermined weight or to a predetermined volume of the sample, whereafter the container volume is closed with the closure. The analytical liquid may be released, and the sample and analytical liquid may be mixed, for instance by shaking the closed container, although also other methods known in the art may be applied. In principle, the container may also be equipped with an internal device for mixing, for instance a device that can be stirred by a magnetic field that is applied with an external magnetic field generator. By mixing and homogenizing the liquid and sample, possible present pesticides, or other compounds to be analysed, may solve in the analytical liquid, whereby the compounds to be analysed are "conserved". The analytical liquid is added in a predetermined volume. In this way, a reliable amount of sample and a reliable amount of analytical liquid are combined, which allows a standard determination later. The container can, if desired, be shipped to a specialized laboratory, whereas possible decay of the sample may be of no influence on the solved pesticide(s) or other contaminant(s). Further, a much smaller sample can be sent to the specialized laboratory. Would in the past a large sample, such a kg or more fruit, etc. be shipped to the specialized laboratory, now only a small flask can be shipped, which may reduce costs and/or which may reduce time.

The method may especially be applied to determine pesticides. Hence, in a specific embodiment, the analytical liquid comprises one or more pesticide residue analysis solvents selected from the group consisting of acetone, acetonitril, ethyl acetate, or any other commonly used solvents for the analyses of pesticide residues. Further, the analytical liquid may comprise an internal standard, such as known in the art. The method may also be applied to determine other contaminants, such as selected from the group consisting of hormones, pharmaceuticals for humans, pharmaceuticals for animals, etc. In general, the analytical liquid comprises one or more solvents selected from the group consisting of acetone, acetonitril, ethyl acetate, or any other commonly used solvents for the analyses of the contaminant(s) to be analysed.

In a specific embodiment, the closed compartment is an ampoule. Such ampoule may for instance be of glass. The ampoule may for instance be broken or crushed when closing the container volume with the closure. For instance, by turning a closure on a container, at the same time, pressure may be applied to the closed compartment, such as an ampoule. Since breaking or crushing may lead to pieces of material of the closed compartment, it may be desired to arrange the closed compartment behind a filter, such as a sieve. This filter is permeable for the analytical liquid, but may reduce or prevent pieces of the closed compartment after opening entering the container volume from behind the filter. Hence, in an embodiment, the closed compartment, such as the ampoule, is configured to allow providing the analytical liquid to the container volume while arranging the closure to the container volume. In yet another embodiment, the closed compartment, such as the ampoule, is integrated in the closure for closing the container volume. The term closed compartment and ampoule may also in embodiments refer to a plurality of closed compartments and ampoules, respectively.

The term "upon activation" may for instance include pressure via a device when screwing or pressing the closure to the container.

Optionally, the closed compartment, such as the ampoule, may be configured to allow providing the analytical liquid to the container volume after arranging the closure to the container volume. For instance, the container may comprise means that allow providing pressure ("activation") from external to part of the container volume, such as in an embodiment a penetration device, that may be used to press or scratch the closed compartment until opening. Alternatively, the closed compartment may include a compartment hull of which at least part solvable in water and/or degrades in the presence of acids ("activation"). In this way, with time, the closed compartment opens. Then, mixing and homogenizing may take place (see also above). Alternatively or additionally, the compartment may be configured to open or brake upon (vigorously) shaking or moving the container, such as in an embodiment after filling with the sample and closing the container volume with the closure. In principle, the closed compartment may also comprise an electrically conductive wire that can heat part of the compartment until opening thereof, under influence of an electric field.

After having added the (mashed) sample to the container volume, adding the analysis liquid, and mixing the sample an analysis liquid, the liquid in the container can be analysed.

For instance, this may be performed after an optional shipping, and/or after an optional further homogenizing. For instance, in an embodiment (for instance after transfer to a laboratory), the closure is removed and the container is arranged in or attached to an apparatus for further homogenization. Optionally, all material from the container can be removed and can in an apparatus for further homogenization further be homogenized.

Thereafter, at least part of the liquid may be removed and may be analysed on the presence, and if present, on the type, and optionally also on the amount (if any) of one or more pesticides or other contaminants, if desired. Hence, in a specific embodiment, the invention also provides a method for preparing a sample, such as a sample selected from the group consisting of a fruit sample, a vegetable sample, a water sample, a soil sample, or a plant material sample, for analysis, the method comprising:
a. providing a sample to a container volume of a container, wherein the container volume or a closure therefore comprise a closed compartment containing an analytical liquid, wherein the closed compartment is configured to provide the analytical liquid into the container volume upon activation;
b. closing the container volume with the closure;
c. opening the closed compartment and mixing the sample and the analytical liquid; and
d. analysing at least part of the mixture of sample and analytical liquid.

Analysis may refer to any analysis method that can start, optionally after further preparation, from a solved analyte, such as a pesticide residue, as starting material. Especially GC or LC, such as GC-MS or LC-MS, may be of relevance. Other methods that may be applied are GC or LC with for instance IT, MS/MS, TOF, ECD, FID detection. Other methods known in the art may also be applied.

In an embodiment, the container is a double-wall container, wherein the container is suitable to host a cooling medium in the volume between the walls of the double wall. For instance, the cooling medium can be a liquid or a gel. Preferably, the sample is maintained at a temperature of 7 °C or lower, more preferably 5 °C or lower, such as in the range of -20-5 °C (i.e. from minus 20 up to 5 °C). Hence, the invention also provides a method of transporting a prepared vegetable sample, especially a prepared fruit sample, the method comprising arranging the container with the ground vegetable sample, such as a mashed fruit sample, and analytical liquid in a transport unit, and transporting the transport unit while maintaining the temperature of the container at 5 °C or lower.

In a further aspect, the invention provides a combination of a container, having a container volume, and a closure therefore, wherein the container volume or the closure comprise a closed compartment containing an analytical liquid, wherein the closed compartment is configured to provide the analytical liquid into the container volume upon activation, wherein the analytical liquid comprises one or more (pesticide residue) analysis solvents selected from the group consisting of acetone, acetonitril, ethyl acetate, or other commonly used solvents for the analyses of pesticide residues (or other contaminant(s)), and wherein the analytical liquid optionally comprises an internal standard.

The closed compartment may for instance be an ampoule, see also above.

In an embodiment, the closed compartment, such as the ampoule, may be integrated in a closure for closing the container volume. Especially, the closed compartment, such as the ampoule, is configured to allow providing the analytical liquid to the container volume while arranging the closure to the container volume. In another embodiment, the ampoule is configured to allow providing the analytical liquid to the container volume after arranging the closure to the container volume.

In yet a further embodiment, the closed compartment is arranged behind a filter, such as a sieve, thereby providing a closed compartment separated from the container volume by a filter. The filter may especially be configured to pass the analytical liquid to the container volume, but reduce or prevent migration from particles from the closed compartment, such as glass pieces when using a glass ampoule as closed compartment for the analytical liquid.

According to another aspect, the invention provides a container comprising a with a closure closed container volume, the container volume containing a mixture of a sample, such as selected from the group consisting of a fruit sample, a vegetable sample, a water sample, a soil sample, or a plant material sample, especially a ground vegetable sample, and an analytical liquid, (i.e. a prepared sample), wherein the analytical liquid comprises one or more solvents selected from the group consisting of acetone, acetonitril, ethyl acetate, or other commonly used solvents for the analyses of pesticide residues or other contaminant(s), and wherein the analytical liquid optionally comprises an internal standard. Such filled and closed container may for instance be shipped to a dedicated analysis laboratory.

According to again another aspect, the invention provides a method for analysing a sample, preferably selected from the group consisting of a fruit sample, a vegetable sample, a water sample, a soil sample, or a plant material sample, especially a mashed fruit sample, comprising analysing at least part of a mixture of the, optionally mashed, (fruit) sample and the analytical liquid from a container volume obtainable by the method as defined herein.

According to yet another aspect, the invention provides a kit comprising (a) combination of container and a closure therefore as defined herein, (b) a cleaning liquid for cleaning at least part of the container volume, (c) a grinder or blender, (d) optionally one or more selected from the group consisting of a knife and a spoon, (f) optionally a manual for sampling and sample preparation instructions, and (g) optionally packaging material for packaging the filled container. Optionally, the packaging material facilitates providing a container compartment for hosting the closed container and facilitates providing a cooling medium compartment configured to cool the container. For instance, the packaging material may be of (polystyrene) foam, having a cut-away for the closed container, and a second cut-away, configured to at least partly surround the cut-away for the closed container, and configured to host a cooling medium. This may especially be relevant when transporting the container. In an embodiment, the cooling medium comprises a cooling gel.

The term "combination of container and a closure" may also refer to a plurality of containers and closures, respectively.

The packaging material may for instance be used to facilitate transportation (such as international transport by plane) of the container containing the mixture of the ground vegetable sample, such as a mashed (fruit) sample, and the analytical liquid without leakage.

In an embodiment, the invention further provides a transport unit, comprising a transport unit volume with cooling jacket. The cooling jacket may be used to cool the combination of container and container closure. The cooling jacket may be cooled by an electronic cooling element, but the cooling jacket may also cool by containing a cooling liquid or a cooling gel. The cooling jacket may be rigid, but the cooling jacket may in an embodiment also be flexible. In the latter embodiment, the cooling jacked may be folded around the container.

Hence, especially a transport unit is provided, comprising a transport unit volume for hosting (a) the combination of container and container closure (as described above or below), and (b) a cooling jacket configured to surround at least part of the container. The cooling jacket may be in physical contact with the container, but in an embodiment, the container is arranged in a metal cylinder. Such cylinder may be used to aid thermal transport between container and cooling jacket, and may further be used a protective means. The cooling jacket may be configured to surround at least part of the container (optionally with the cylinder between container and cooling jacket), but the cooling jacket may optionally also be configured to surround at least part of the closure. Preferably, the sample is maintained at a temperature of 7 °C or lower, more preferably 5 °C or lower, such as in the range of -20-5 °C. Hence, the invention also provides a method of transporting a prepared fruit sample, the method comprising arranging the container with vegetable sample, such as a (mashed) fruit sample, and analytical liquid as described above or below in the transport unit (i.e. in the transport unit volume), and transporting the transport unit while maintaining the temperature of the cooling jacket at 7 °C or lower, more preferably at 5 °C or lower. By maintaining the cooling jacket at this temperature, the container may be maintained at this temperature, i.e. the vegetable sample, such as the (mashed) fruit sample, (and analytical liquid) is maintained at such temperature.

### Specific embodiment without compartment for analytical liquid

In yet another embodiment, the invention provides a method for preparing a vegetable sample for analysis, comprising (a) providing a ground vegetable sample to a container volume of a container, (b) mixing the vegetable sample and the analytical liquid (such as defined above), and (c) closing the container volume with a closure. In this method embodiment, the container or closure do not necessarily comprise the above described compartment. Such container with vegetable sample, such as a fruit sample or a sample of a vegetable (such as lettuce, etc.), mixed with analytical liquid, obtained by such method, may for instance be transported with the above indicated transport unit. As indicated above, the analytical liquid may comprise one or more pesticide residue analysis solvents selected from the group consisting of acetone, acetonitril, ethyl acetate, and wherein optionally the analytical liquid comprises an internal standard.

Hence, in a specific embodiment, the invention also provides a container comprising a with a closure closed container volume, the container volume containing a mixture of a ground vegetable sample and an analytical liquid, wherein the analytical liquid comprises one or more pesticide residue analysis solvents selected from the group consisting of acetone, acetonitril, ethyl acetate, and wherein optionally the analytical liquid comprises an internal standard. In this container with vegetable sample, the container or closure do not necessarily comprise the above described compartment. Such container with vegetable sample mixed with analytical liquid (i.e. prepared sample) may for instance be transported with the above indicated transport unit.

In a further embodiment, the invention also provides a method for analysing a vegetable sample, such as especially a fruit sample, comprising analysing at least part of a mixture of a ground vegetable sample and an analytical liquid from a container volume obtainable by the method described above.

In yet a further aspect, the invention provides a kit comprising a combination of container and a closure therefore as described above, a cleaning liquid for cleaning at least part of the container volume; and, a grinder or blender, optionally one or more selected from the group consisting of a knife and a spoon, optionally a manual for sampling and sample preparation instructions, and optionally packaging material for packaging the filled container.

In yet a further aspect, the invention also provides a method of transporting a prepared vegetable sample, the method comprising arranging the container with ground vegetable sample and analytical liquid (such as described herein) in the transport unit as described herein, and transporting the transport unit while maintaining the temperature of the cooling jacket at 5 °C or lower.

### Brief description of the drawings

Embodiments of the invention will now be described, by way of example only, with reference to the accompanying schematic drawings in which corresponding reference symbols indicate corresponding parts, and in which:
Figure 1 schematically depicts a container with closure;
Figures 2a-2c schematically depict some possible embodiments of the closure and container with closure; Figures 3a-3b schematically depict some principles of the invention;
Figure 4 schematically depicts an embodiment of the transport unit The figures are not necessarily on scale.

### Description of preferred embodiments

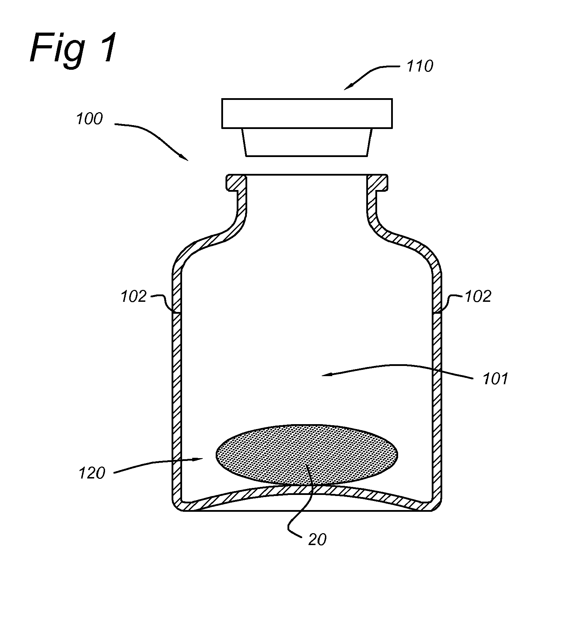

Figure 1 schematically depicts an embodiment of a combination of a container 100, having a container volume 101, and a closure 110 therefore. The container 100 or the closure 110 comprise a closed compartment 120 containing an analytical liquid 20. The closed compartment 120 is configured to provide the analytical liquid into the container volume 101 upon activation. Here, the closed compartment 120 is arranged in the container 100; in the schematic drawing effectively in the container volume 101. The container 100 comprises by way of example one or more indication bars 102 to indicate to the use to which level the container volume has to be filled with sample, such as a mashed apple sample or a mashed banana sample.

In this and other embodiments described herein, the ratio of the volume for the sample (not indicated) and the volume for the analytical liquid may be any workable ratio, but may especially be in the range of 5:1 - 1:5, such as 2:1-1:2.

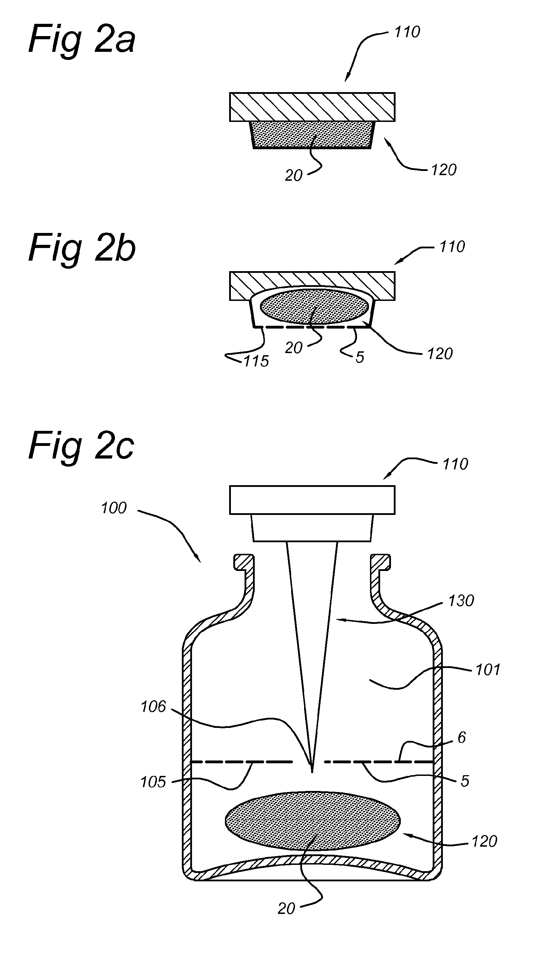

Figure 2a schematically depicts a closure 100, wherein this closure 100 comprises the closed compartment 120. This closed compartment 120 can be prefilled with a predetermined amount of the analysis liquid.

Figure 2b schematically depicts another embodiment of the closure 100, wherein the closed compartment 120 is integrated in the closure 110. Here, the closed compartment 120 is provided as ampoule. The ampoule is arranged between a filter 5, such as a sieve, which is (thus) here a closure filter, indicated with reference 115. Especially, the filter is configured to, in a state wherein the container 100 is closed with the closure 110 and the closed compartment 120 is not yet opened, be between the container volume 101 and the closed compartment 120.

Figure 2c schematically depicts an embodiment of a device, opening device 130, that can be used to open the closed compartment 120 upon external action, such as force. Here, the opening device 130 comprises a sharp item that is attached to the closure 110 and that, when arranging the closure 110 to the container penetrates or breaks the closed compartment 120. In this way, analytical liquid may escape and can be mixed with the sample (not indicated). Reference 105 refers to the filter 5 being arranged in the container volume 101; likewise, reference 106 refers to opening 6 of the filter 105.

Herein, the closure 110 can in principle be any closure able to close the container volume 101 in a state substantially preventing escape or entrance from liquid or gas from or into the container volume 101.

The closure 110 may be a closure configured to be rotateably arranged to the container 100 when closing, or a closure configured to be slideably arranged to the container 100, when closing, etc.

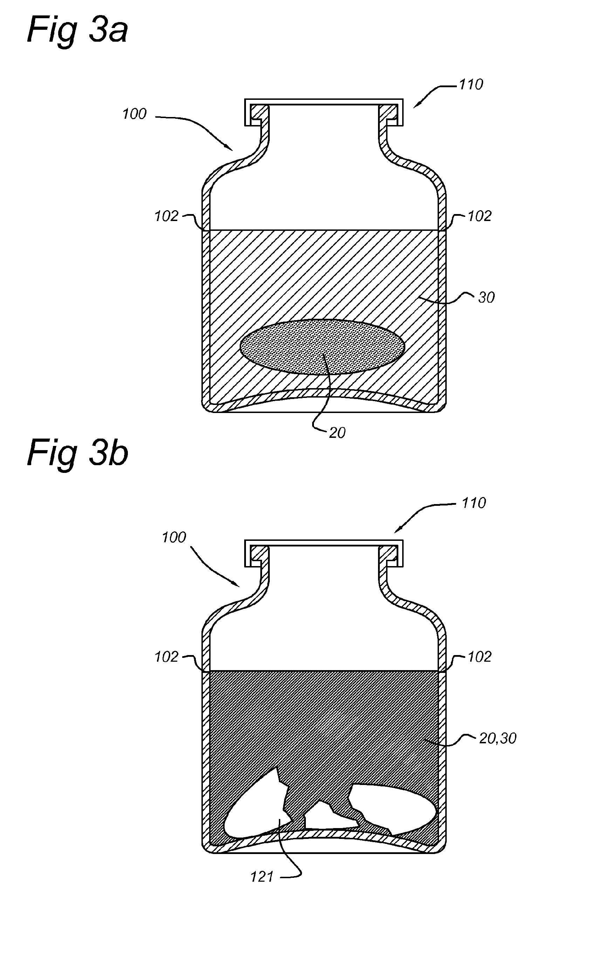

Figures 3a and 3b show a (nearly) closed state and a closed state wherein the closed compartment 120 is (still) closed or has been opened, respectively. Here, by way of example, another type of closure 110 is depicted. The sample, such as a mashed fruit sample, is indicated with reference 30. Upon opening the closed compartment 120, for instance by the methods described above, the analytical liquid is released and can mix with the sample 30. Homogenisation may be promoted by shaking. By way of example, in figure 3b compartment pieces 121 are indicated.

The material of the container 100 and the closure 110 may be any material, but may especially be a plastic, even more especially be HDPE (high density polyethylene). The closed compartment 120 (or more especially its wall) may comprise any material, but preferably a material that can be broken under pressure, such as glass, or may at least partly comprise a material that solves upon contact with water and/or acids (but does not solve in the pesticide residue analysis solvent(s).

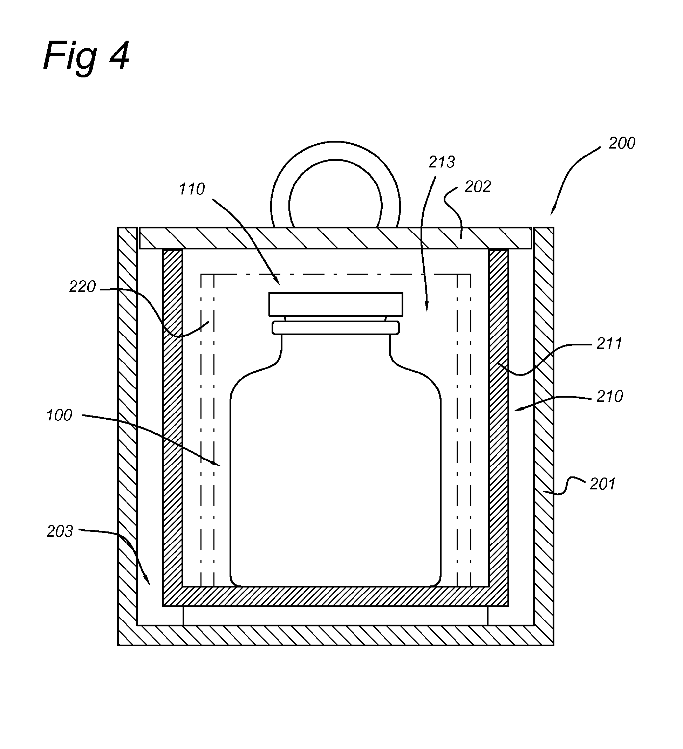

Figure 4 schematically depicts an embodiment of a transport unit 200. The transport unit 200 comprises a transport unit volume 203 for hosting the combination of container 100 and container closure 110 (as described above) and (b) a cooling jacket 210 configured to surround at least part of the container 100. The transport unit 200 comprises a wall 201, providing the transport unit volume 203. Further, the transport unit 200 may comprise a transport unit closure 202. The cooling jacket 210 may be cylindrical, with or without bottom. The cooling jacket 210 may also consist of several parts. In this schematically depicted embodiment, the cooling jacket 210 has a cylindrical shape, including a bottom, providing a jacket volume 213. The jacket volume 213 is configured to host the container 100 (and container closure 110). In the schematically depicted embodiment, the container 100 is arranged in a cylinder 220, which is optional, and the cylinder 220 and container 100 are arranged in the jacket volume 213. The cooling jacket 210 may for instance host a cooling liquid or cooling gel, indicated with reference 211. The whole transport unit 200 including closure 202 may be transported and may effectively cool the sample in the container 100. In this way, the sample (in the container 100) may be maintained at a temperature of for instance 5 °C or lower, such as in the range of -20-5 °C.

### Specific embodiments

The invention especially relates to the following embodiments:
1. A method embodiment for preparing a vegetable sample (such as a mashed fruit sample) for analysis, comprising:
   a. providing a ground vegetable sample to a container volume of a container, wherein the container volume or a closure therefore comprise a closed compartment containing an analytical liquid, wherein the closed compartment is configured to provide the analytical liquid into the container volume upon activation;
   b. closing the container volume with the closure; and
   c. opening the closed compartment and mixing the vegetable sample and the analytical liquid.
2. The method according to method embodiment 1, wherein the analytical liquid comprises one or more pesticide residue analysis solvents selected from the group consisting of acetone, acetonitril, ethyl acetate, and wherein the analytical liquid comprises an internal standard.
3. The method according to any one of method embodiments 1-2, wherein the closed compartment is an ampoule.
4. The method according to any one of method embodiments 1-3, wherein the closed compartment is integrated in the closure for closing the container volume.
5. The method according to any one of method embodiments 1-4, wherein the closed compartment is configured to allow providing the analytical liquid to the container volume while arranging the closure to the container volume.
6. The method according to any one of method embodiments 1-4, wherein the closed compartment is configured to allow providing the analytical liquid to the container volume after arranging the closure to the container volume.
7. The method according to any one of the preceding method embodiments, wherein the container volume or a closure further comprises a filter, configured to, in a state wherein the container is closed and the closed compartment is not yet opened, be between the container volume and the closed compartment.
8. The method according to any one of the preceding method embodiments, further comprising analysing at least part of the mixture of ground vegetable sample and analytical liquid.
9. A combination of a container, having a container volume, and a closure therefore, wherein the container volume or the closure comprise a closed compartment containing an analytical liquid, wherein the closed compartment is configured to provide the analytical liquid into the container volume upon activation, wherein the analytical liquid comprises one or more pesticide residue analysis solvents selected from the group consisting of acetone, acetonitril, ethyl acetate, and wherein optionally the analytical liquid comprises an internal standard.
10. The combination according to combination embodiment 9, wherein the closed compartment is an ampoule.
11. The combination according to combination embodiment 10, wherein the closed compartment is integrated in a closure for closing the container volume.
12. The combination according to any one of combination embodiments 9-11, wherein the closed compartment is configured to allow providing the analytical liquid to the container volume while arranging the closure to the container volume.
13. The combination according to any one of combination embodiments 9-11, wherein the closed compartment is configured to allow providing the analytical liquid to the container volume after arranging the closure to the container volume.
14. The combination according to any one of combination embodiments 9-13, wherein the container volume or a closure further comprises a filter, configured to, in a state wherein the container is closed and the closed compartment is not yet opened, be between the container volume and the closed compartment.
15. A container comprising a with a closure closed container volume, the container volume containing a mixture of a ground vegetable sample and an analytical liquid, wherein the analytical liquid comprises one or more pesticide residue analysis solvents selected from the group consisting of acetone, acetonitril, ethyl acetate, and wherein optionally the analytical liquid comprises an internal standard.
16. A method for analysing a vegetable sample, comprising analysing at least part of a mixture of a ground vegetable sample and an analytical liquid from a container volume obtainable by the method according to any one of method embodiments 1-7.
17. A kit comprising
   a. a combination of container and a closure therefore according to any one of combination embodiments 9-14;
   b. a cleaning liquid for cleaning at least part of the container volume; and
   c. a grinder or blender;
   d. optionally one or more selected from the group consisting of a knife and a spoon;
   e. optionally a manual for sampling and sample preparation instructions; and
   f. optionally packaging material for packaging the filled container.

The above may also apply to other samples, such as a water sample, a soil sample, or a tissue sample, etc.

The term "substantially" herein, such as in "substantially all emission" or in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. In the device claim enumerating several means, several of these means may be embodied by one and the same item of hardware. The mere fact that certain measures are recited in mutually different dependent claims does not indicate that a combination of these measures cannot be used to advantage.

## Claims

1. A method for preparing a vegetable sample for analysis, comprising:
a. providing a ground vegetable sample to a container volume of a container, wherein the container volume or a closure therefore comprise a closed compartment containing an analytical liquid, wherein the closed compartment is configured to provide the analytical liquid into the container volume upon activation;
b. closing the container volume with the closure; and
c. opening the closed compartment and mixing the ground vegetable sample and the analytical liquid.

2. The method according to claim 1, wherein the analytical liquid comprises one or more pesticide residue analysis solvents selected from the group consisting of acetone, acetonitril, ethyl acetate, and wherein the analytical liquid comprises an internal standard.

3. The method according to any one of claims 1-2, wherein the closed compartment is an ampoule.

4. The method according to any one of claims 1-3, wherein the closed compartment is integrated in the closure for closing the container volume.

5. The method according to any one of claims 1-4, wherein the closed compartment is configured to allow providing the analytical liquid to the container volume while arranging the closure to the container volume or wherein the closed compartment is configured to allow providing the analytical liquid to the container volume after arranging the closure to the container volume.

6. The method according to any one of claims 1-5, wherein the ground vegetable sample is a mashed fruit sample.

7. A combination of a container, having a container volume, and a closure therefore, wherein the container volume or the closure comprise a closed compartment containing an analytical liquid, wherein the closed compartment is configured to provide the analytical liquid into the container volume upon activation, wherein the analytical liquid comprises one or more pesticide residue analysis solvents selected from the group consisting of acetone, acetonitril, ethyl acetate, and wherein optionally the analytical liquid comprises an internal standard.

8. The combination according to claim 7, wherein the closed compartment is an ampoule.

9. The combination according to any one of claims 7-8, wherein the closed compartment is integrated in a closure for closing the container volume.

10. The combination according to any one of claims 7-9, wherein the closed compartment is configured to allow providing the analytical liquid to the container volume while arranging the closure to the container volume or wherein the closed compartment is configured to allow providing the analytical liquid to the container volume after arranging the closure to the container volume.

11. The combination according to any one of claims 7-10, wherein the container volume or a closure further comprise a filter, configured to, in a state wherein the container is closed and the closed compartment is not yet opened, be between the container volume and the closed compartment.

12. A container comprising a with a closure closed container volume, the container volume containing a mixture of a ground vegetable sample and an analytical liquid, wherein the analytical liquid comprises one or more pesticide residue analysis solvents selected from the group consisting of acetone, acetonitril, ethyl acetate, and wherein optionally the analytical liquid comprises an internal standard.

13. A transport unit, comprising a transport unit volume for hosting (a) the combination of container and container closure according to any one of claims 7-11, and (b) a cooling jacket configured to surround at least part of the container.

14. A method for analysing a vegetable sample, comprising analysing at least part of a mixture of a ground vegetable sample and an analytical liquid from a container volume obtainable by the method according to any one of claims 1-6.

15. A method of transporting a prepared vegetable sample, the method comprising arranging the container with ground vegetable sample and analytical liquid according to any one of claims 1-6 in the transport unit according to claim 13, and transporting the transport unit while maintaining the temperature of the cooling jacket at 5 °C or lower.

16. A kit comprising
a. a combination of container and a closure therefore according to any one of claims 7-11;
b. a cleaning liquid for cleaning at least part of the container volume; and
c. a grinder or blender;
d. optionally one or more selected from the group consisting of a knife and a spoon;
e. optionally a manual for sampling and sample preparation instructions; and
f. optionally packaging material for packaging the filled container.
